# EUROPEAN PATENT APPLICATION

(11) **EP 4 059 945 A1**
(43) Date of publication of application: **21.09.2022**
(21) Application number: 20893943.9
(22) Date of filing: 20.07.2020
(51) Int. Cl.: C07K 5/093, A61K 8/64, A61Q 7/00, A61Q 19/00

(54) **PEPTIDES INHIBITING ANDROGEN RECEPTOR ACTIVITY, AND COSMETICS COMPOSITION USING SAME**

(30) Priority: 29.11.2019 KR 20190157560
(71) Applicant: Supadelixir Inc., Chuncheon-si, Gangwon-do 24232 (KR)
(72) Inventor: HAHN, Jang Hee, Chuncheon-si Gangwon-do 24375 (KR); KIM, Min Seo, Chuncheon-si Gangwon-do 24377 (KR)
(74) Representative: González López-Menchero, Álvaro Luis
(86) International application number: PCT/KR2020/009498
(87) International publication number: WO 2021/107316

(57) **Abstract**

Disclosed are peptides and a cosmetics composition using the same peptides. The peptides inhibit the activity of an androgen receptor (AR), whereby the peptides may be used to alleviate androgen-related diseases or disorders such as prostate cancer, skin diseases, and alopecia.

<110>    SUPADELIXIR INC.

<120>    Peptide for inhibiting activity of androgen receptor and Cosmetic
         composition using the same

<130>    PFAHJ-20117

<150>    KR 10-2019-0157560
<151>    2019-11-29

<160>    1

<170>    KoPatentIn 3.0

<210>    1
<211>    3
<212>    PRT
<213>    Artificial Sequence

<220>
<223>    Peptide 1


<400>    1
Asp Lys Phe
  1

## Description

### Technical Field

The present disclosure relates to peptides inhibiting the activity of an androgen receptor (AR) and a cosmetics composition using the same peptides.

### Background Art

Androgens are steroidal hormones known as male sex hormones or testoids. The androgens bind to an androgen receptor (AR), thereby regulating the growth and development of the male reproductive system and performing various functions such as inhibiting fat accumulation and increasing muscle mass. The androgens include testosterone that is secreted in the testicles, androsterone and dehydroepiandrosterone which are converted from testosterone and excreted in urine, and adrenosterone produced in the adrenal cortex.

The androgens act only via the AR that is a protein transcription factor interacting with a specific region of DNA. The mode of action of testosterone and more potent homologue dihydrotestosterone (DHT) thereof is determined depending on binding to the AR, and the transcription by the RNA polymerase II then occurs.

Aside from the physiological regulation, the androgens are associated with many diseases. For example, it is known that early progression of prostate cancer is attributable to androgens, acne occurs due to an inflammatory reponse when androgens irritate sebaceous glands to produce an excess amount of sebum, and hair loss occurs due to hair follicle destroying substances, such as BMP, DKK-1, and TGF-β1, which are secreted when denatured androgens such as DHT bind to the AR. For this reason, androgen receptor activity suppressors that inhibit the activity of the AR have been developed to treat diseases induced by androgens as disclosed in literatures listed below.

### <Patent Literature>

Korean Patent Application Publication No. 10-2002-0089347 (published on November 29, 2002) "Androgen Receptor Suppressors in the Therapy and Diagnosis of Prostate Cancer, Alopecia, and Other Hyper-androgenic Syndromes".

However, the existing androgen receptor suppressors are not free from side effects when used for the treatment of androgen-related diseases because they are mostly chemically synthesized.

### Disclosure

### Technical Problem

The present disclosure has been made keeping in mind the problems occurring in the related art.

An objective of the present disclosure is to provide a peptide inhibiting the activity of an androgen receptor (AR), and a cosmetics composition using the same, the peptide and the cosmetics composition being used to alleviate androgen-related diseases or disorders such as prostate cancer, skin diseases, and alopecia.

### Technical Solution

The present disclosure is implemented according to embodiments having the following constructions to accomplish the above objective.

According to one embodiment of the disclosure, there is provided a peptide including the amino acid sequence of SEQ ID NO: 1.

According to another embodiment of the disclosure, all of the amino acids constituting the peptide may be D-amino acids, and the peptide reduces an activity of an androgen receptor (AR).

According to a further embodiment of the disclosure, the peptide may be used to alleviate skin troubles.

According to a yet further embodiment of the disclosure, the peptide may be used to alleviate acne or alopecia.

According to a yet further embodiment of the disclosure, there is provided a cosmetics composition including a peptide composed of the amino acid sequence of SEQ ID NO: 1, and all of the amino acids constituting the peptide may be D-amino acids.

According to a yet further embodiment of the disclosure, the cosmetics composition may have an effect of alleviating acne or alopecia.

### Advantageous Effects

According to the above-described embodiments, the present disclosure has advantages described below.

The present disclosure uses the peptide including the amino acid sequence of SEQ ID NO: 1 to inhibit the activity of androgen receptors, thereby alleviating androgen-related diseases or disorders, such as prostate cancer, skin diseases, and alopecia.

### Description of Drawings

FIG. 1 is an image illustrating the results of Western blot analysis performed to check the effect of the peptide according to an embodiment of the present disclosure on the PSA expression; and
FIG. 2 is a graph showing the impact of the peptide according to an embodiment of the present disclosure on physical binding of AR and POL II.

### Best Mode

Hereinafter, peptides inhibiting the activity of an androgen receptor (AR) and a cosmetics composition using the same according to preferred embodiments of the present disclosure will be described in detail with reference to the accompanying drawings. Unless not specifically defined, all terminologies in the specification should be interpreted based on the general meanings thereof that a person skilled in the art understands. When the general meanings of the terminologies are incompliant with those used in the specification, the terminologies should be interpreted as being defined herein. In describing the present disclosure, well-known functions or constructions will not be described in detail in case they may unnecessarily obscure the understanding of the present disclosure. Also, when a part "includes" or "comprises" an element in the description, unless there is a particular description contrary thereto, the part can further include other elements, not excluding the other elements.

In the present disclosure, an embodiment relates to peptides inhibiting AR activity. The peptides includes the amino acid sequence of SEQ ID NO: 1 (Asp-Lys-Phe), and all of the amino acids (Asp, Lys, and Phe) constituting the peptides are D-amino acids. The peptides inhibit the AR activity, thereby alleviating diseases caused by androgens binding to ARs. Specifically, the peptides may alleviate prostate cancer and skin diseases by suppressing the AR activity. More specifically, the peptides may alleviate acne by preventing an excessive production of sebum and may reduce DHT-induced hair loss.

In the present disclosure, another embodiment relates to a cosmetics composition including a peptide including the amino acid sequence of SEQ ID NO: 1 (Asp-Lys-Phe), and all of the amino acids constituting the peptide are D-amino acids. The cosmetics composition may alleviate skin troubles such as acne or hair loss.

The cosmetics composition may further include one or more components selected from the group consisting of water-soluble vitamins, oil-soluble vitamins, high molecular polysaccharides, sphingolipids, and seaweed extracts.

As for the water-soluble vitamins, anything that can be blended in cosmetics can be used. Preferably, examples thereof include vitamin B1, vitamin B2, vitamin B6, pyridoxine, pyridoxine hydrochloride, vitamin B12, pantothenic acid, nicotinic acid, nicotinic acid amide, folic acid, vitamin C, and vitamin H. However, salts of the examples, such as thiamine hydrochloride, sodium ascorbate, and derivatives thereof, such as ascorbic acid-2-phosphate sodium salt, and ascorbic acid-2-phosphate magnesium salt may also be included in the examples of the water-soluble vitamins to be used in the embodiment. The water-soluble vitamins may be obtained by conventional methods such as microbial transformation, purification from microbial culture, enzymatic method, and synthesizing method.

As for the oil-soluble vitamins, anything that can be blended in cosmetics can be used. Preferably, examples thereof include vitamin A, carotin, vitamin D2, vitamin D3, and vitamin E (d1-alpha tocopherol, d-alpha tocopherol, d-alpha tocopherol). However, derivatives thereof, such as ascorbine palmitate, ascorbine stearate, ascorbine dipalmitate, dl-alpha tocopherol acetate, nicotinic acid dl-alpha tocopherol vitamin E, DL-pantothenyl alcohol, D-pantothenyl alcohol, and pantothenyl ethyl ether may also be included in the examples of the oil-soluble vitamins to be used in the embodiment. The oil-soluble vitamins may be obtained by conventional methods such as microbial transformation, purification from microbial culture, an enzymatic method, and a synthesizing method.

As for the high molecular polysaccharides, anything that can be blended in cosmetics can be used. Preferably, examples thereof include hydroxyethyl cellulose, xanthan gum, sodium hyaluronate, and chondroitin sulfate or its salt form (sodium salt). For example, chondroitin sulfate or its salt form can be mainly extracted and purified from sources like mammals and fish.

As for the sphingolipids, anything that can be blended in cosmetics can be used. Preferably, examples thereof include ceramide, phytosphingosine, and sphingoglycolipids. The sphingolipids may be obtained by purifying an extract of mammals, fish, shellfish, yeast, or plants, or by chemical synthesis.

As for the seaweed extracts, anything that can be blended in cosmetics can be used. Preferably, examples thereof include brown algae extract, red algae extract, and green algae extract. However, their purified extracts such as calrageenan, arginic acid, sodium alginate, and potassium arginate may also be included as seaweed extracts to be used in the embodiment. The seaweed extracts can be extracted and purified from seaweeds by conventional methods.

The cosmetics composition in the present disclosure may include other ingredients that are commonly used for cosmetic compositions. For example, one or more ingredients selected from among oil and fat ingredients, moisturizing agents, emollients, surfactants, organic and inorganic pigments, organic powders, ultraviolet absorbers, preservatives, fungicides, antioxidants, plant extracts, pH adjusters, alcohols, pigments, fragrances, blood circulation promoters, cooling agents, antiperspirants, and purified water may be further included.

The oil and fat ingredients include an ester oil, a hydrocarbon oil, silicone grease, fluorine-based fats, animal fats, and plant fats. Examples of the ester oil include glyceryl tri(2-ethylhexanoate), cetyl 2-ethylhexanoate, isopropyl myristate, butyl myristate, isopropyl palmitate, ethyl stearate, octyl palmitate, isocetyl isostearate, butyl stearate, ethyl linoleate, isopropyl linoleate, ethyl oleate, isocetyl myristate, isostearyl myristate, isostearyl palmitate, octyldodecyl myristate, isocetyl isostearate, diethyl sebacate, diisopropyl adipate, isoalkyl neopentanoate, tri(caprylic acid, capric acid) glyceryl, trimethylolpropane 2-ethylhexanoate, trimethylol propane triisostearate, pentaelysitol tetra(2-ethylhexanoate), cetyl caprylate, decyl laurate, hexyl laurate, myristic acid decyl, myristyl myristate, cetyl myristate, stearyl stearate, decyl oleate, cetyl ricinoleate, isostearyl laurate, isotridecyl myristate, isocetyl palmitate, octyl stearate, isocetyl stearate, isodecyl oleate, octyldodecyl oleate, octyldodecyl linoleate, isopropyl isostearate, cetostearyl 2-ethylhexanoate, stearyl 2-ethylhexanoate, hexyl isostearate, ethylene glycol dioctanoate, ethylene glycol dioleate, propylene glycol dicapric acid, propylene glycol di(caprylic, capric acid), propylene glycol dicaprylate, neopentyl glycol dicapric acid, neopentyl glycol dioctanoate, glyceryl tricaprylate, glyceryl triundecyl acid, glyceryl triisopalmitate, glyceryl triisostearate, octyldodecyl neopentanoate, isostearyl octanoate, octyl isononanoate, hexyldecyl neodecanoate, octyldodecyl neodecanoate, isocetyl isostearate, isostearyl isostearate, octyldecyl isostearate, polyglycerol oleic acid ester, polyglycerol isostearic acid ester, triisocetyl citrate, triisoalkyl citrate, triisooctyl citrate, lauryl lactate, myristyl lactate, cetyl lactate, octyldecyl lactate, triethyl citrate, acetyl triethyl citrate, acetyl tributyl citrate, trioctyl citrate, diisostearyl malate, 2-ethylhexyl hydroxystearate, di2-ethylhexyl succinate, diisobutyl adipate, diisopropyl sebacate, dioctyl sebacate, cholesteryl stearate, cholesteryl isostearate, cholesteryl hydroxystearate, cholesteryl oleate, dihydrocholesteryl oleate, phytsteryl isostearate, phytsteryl oleate, 12-stealoylhydroxystearate isocetyl, 12-stealoylhydroxystearate stearate, and 12-stealoylhydroxystearate isostearyl. The hydrocarbon oil may include squalene, liquid paraffin, alpha-olefin oligomer, isoparaffin, ceresin, paraffin, liquid isoparaffin, polybudene, microcrystalline wax, and petrolatum. The silicone grease may include polymethylsilicone, methylphenylsilicone, methylcyclopolysiloxane, octamethylpolysiloxane, decamethylpolysiloxane, dodecamethylcyclosiloxane, dimethylsiloxane-methylcetyloxysiloxane copolymer, dimethylsiloxane-methylstealloxysiloxane copolymer, alkyl-modified silicone oil, and amino-modified silicone oil. The fluorine-based fats may include perfluoropolyether. The animal and plant fats may include avocado oil, almond oil, olive oil, sesame oil, rice bran oil, bird flower oil, soybean oil, corn oil, rapeseed oil, passerin oil, palm kernel oil, palm oil, castor oil, sunflower oil, grape seed oil, cottonseed oil, palm oil, kukui nut Oil, wheat germ oil, rice germ oil, shea butter, moonflower colostrum, marker damia nut oil, meadowfoam oil, egg yolk oil, tallow, horse oil, mink oil, orange raffia oil, jojoba oil, candelabra wax, carnauba wax, liquid lanolin, and hydrogenated castor oil.

The moisturizing agents may include a water-soluble low-molecular moisturizer, a fat-soluble molecular moisturizer, a water-soluble polymer, and a fat-soluble polymer. The water-soluble low-molecular moisturizer may include serine, glutamine, sorbitol, mannitol, pyrrolidone sodium carboxylate, glycerin, propylene glycol, 1,3-butylene glycol, ethylene glycol, polyethylene glycol B (polymerization degree n = 2 or more), polypropylene glycol(polymerization degree n = 2 or more), polyglycerin B(polymerization degree n = 2 or more), lactic acid, and lactate. The fat-soluble molecular moisturizer may include cholesterol and cholesterol ester. The water-soluble polymer may include carboxyvinyl polymer, polyaspartate, tragacanth, xanthan gum, methyl cellulose, hydroxymethylcellulose, hydroxyethylcellulose, hydroxypropylcellulose, carboxymethylcellulose, water soluble chitin, chitosan and dextrin. The fat-soluble polymer may include polyvinylpyrrolidone-eicocene copolymer, polyvinylpyrrolidone-hexadecene copolymer, nitrocellulose, dextrin fatty acid ester, and high molecular weight silicone.

The emollients may include long chain acyl glutamic acid cholesteryl ester, cholesteryl hydroxystearate, 12-hydroxystearic acid, stearic acid, rosin acid, and lanolin fatty acid cholesteryl ester.

The surfactants may include nonionic surfactants, anionic surfactants, cationic surfactants, amphoteric surfactants. The nonionic surfactants may include self-emulsifying glycerin monostearate, propylene glycol fatty acid ester, glycerin fatty acid ester, polyglycerol fatty acid ester, sorbitan fatty acid ester, POE(polyoxyethylene), sorbitan fatty acid ester, POE sorbit fatty acid ester, POE glycerin fatty acid ester, POE alkyl ether, POE fatty acid ester, POE hydrogenated castor oil, POE castor oil, POEPOP(polyoxyethylene polyoxypropylene) copolymer, POEPOP alkyl ether, polyether-modified silicone, lauric acid alkanolamide, alkylamine oxide, and hydrogenated soybean phospholipid. The anionic surfactants may include fatty acid soap, alpha-acylsulfonate, alkylsulfonate, alkylallylsulfonate, alkylnaphthalenesulfonate, alkylsulfate, POE alkyl ether sulfate, alkylamide sulfate, alkyl phosphate, POE alkyl phosphate, alkylamide phosphate, alkyloylalkyltaurine salt, N-acylamino acid salt, POE alkyl ether carboxylate, alkyl sulfosuccinate, sodium alkyl sulfoacetate, acylated hydrolyzed collagen peptide salt, and perfluoroalkyl phosphate ester. The cationic surfactants may include alkyltrimethylammonium chloride, stearyltrimethylammonium chloride, stearyltrimethylammonium bromide, cetostearyltrimethylammonium chloride, distearyldimethylammonium chloride, stearyldimethylbenzylammonium chloride, behenyltrimethylammonium bromide, benzalkonium chloride, diethylaminoethyl stearate, stearic acid dimethylaminopropylamide, and lanolin derivative quaternary ammonium salt. The amphoteric surfactants may include carboxybetaine type, amidebetaine type, sulfobetaine type, hydroxysulfobetaine type, amidesulfobetaine type, phosphobetaine type, aminocarboxylate type, imidazoline derivative type, and amidamine type.

The organic and inorganic pigments may include: the inorganic pigments such as silicic acid, silicic anhydride, magnesium silicate, talc, sericite, mica, kaolin, bengala, clay, bentonite, titanium coated mica, bismuth oxychloride, zirconium oxide, magnesium oxide, zinc oxide, titanium oxide, aluminum oxide, calcium sulfate, barium sulfate, magnesium sulfate, calcium carbonate, magnesium carbonate, iron oxide, ultramarine blue, chromium oxide, chromium hydroxide, calamine and their compounds; and the organic pigments such as polyamide, polyester, polypropylene, polystyrene, polyurethane, vinyl resin, urea resin, phenolic resin, fluororesin, silicon resin, acrylic resin, melamine resin, epoxy resin, polycarbonate resin, styrene-divinylbenzene copolymer, silk powder, cellulose, CI pigment yellow, and CI pigment orange; and organic and inorganic composite pigments.

The organic powders may include: metal soap such as calcium stearate; alkyl phosphate metal salt such as sodium zinc cetylate, zinc laurylate and calcium laurylate; acylamino acid polyvalent metal salt such as N-lauroyl-Beta-alanine calcium, N-lauroyl-Beta-alanine zinc, and N-lauroyl glycine calcium; amidesulfonic acid polyvalent metal salt such as N-lauroyl-taurine calcium and N-palmitoyl-taurine calcium; N-acyl basic amino acid such as N-epsilon-lauroyl-L-lysine, N-epsilon-palmitoylizine, N-alpha-paritoylolnithine, N-alpha-lauroylarginine, and N-alpha-hydrogenated beef fatty acid acylarginine; N-acyl polypeptide such as N-lauroylglycylglycine; alpha-amino fatty acids such as alpha-aminocaprylic acid and alpha-aminolauric acid; and polyethylene, polypropylene, nylon, polymethyl methacrylate, polystyrene, styrene-divinylbenzene copolymer, and ethylene tetrafluoride.

The ultraviolet absorbers may include: para-aminobenzoic acid, ethyl para-aminobenzoate, amyl para-aminobenzoate, octyl para-aminobenzoate, ethylene glycol salicylate, phenyl salicylate, octyl salicylate, benzyl salicylate, butylphenyl salicylate, homomentyl salicylate, benzyl cinnamate, paramethoxycinnamic acid-2-ethoxyethyl, octyl para methoxycinnamate, dipara-methoxycinnamic acid mono-2-ethylhexaneglyceryl, isopropyl paramethoxycinnamate, diisopropyl diisopropyl cinnamic acid ester mixture, urocanic acid, ethyl urokanate, hydroxymethoxybenzophenone, hydroxymethoxybenzophenonesulfonic acid and their salt form; and dihydroxymethoxybenzophenone, sodium dihydroxymethoxybenzophenonedisulfonate, dihydroxybenzophenone, tetrahydroxybenzophenone, 4-tert-butyl-4'-methoxydibenzoylmethane, 2,4,6-trianilino-p-(carbo-2'-ethylhexyl-1'-oxy)-1,3,5-triazine, and 2-(2-hydroxy-5-methylphenyl)benzotriazole.

The fungicides may include hinokitiol, triclosan, trichlorohydroxydiphenyl ether, chlorhexidine gluconate, phenoxyethanol, resorcin, isopropylmethylphenol, azulene, salicylic acid, zinc phyllithione, benzalkonium chloride, photosensitizer No. 301, mononitroguarechol sodium, and undecyrenic acid.

The antioxidants may include butylhydroxyanisole, propyl gallic acid, and elisorbic acid.

The pH adjusters may include citric acid, sodium citrate, malic acid, sodium malate, fmalic acid, sodium fumarate, succinic acid, sodium succinate, sodium hydroxide, and sodium monohydrogen phosphate.

The alcohols may include a higher alcohol such as cetyl alcohol.

Blending ingredients are not limited to the ones mentioned above, and any above-mentioned ingredient may be used for blending within the scope of not damaging the purpose and effect of the present disclosure. However, it would be preferable that the ingredient be blended at a blending ratio of 0.01% to 5% by weight, more preferably 0.01% to 3% by weight relative to the total weight of the cosmetics composition.

The cosmetics composition of the embodiment may take the form of solutions, emulsions, or viscous mixtures.

The cosmetics composition of the embodiment includes the ingredients described above as active ingredients but may also include other ingredients that are commonly used for cosmetics. For example, adjuvants and carriers such as stabilizers, solubilizers, vitamins, pigments, and fragrances may be included.

The cosmetics composition of the embodiment may be produced in any form of formulations that is conventionally used in the art. For example, it may be formulated into emulsions, creams, foundations, lotions, serums, or hair cosmetics. Specifically, it may be formulated into skin lotion, skin softener, moisture lotion, nourishing lotion, massage cream, nourishing cream, hand cream, moisture cream, essence, a pack, soap, lotion, milk lotion, gel, ointment, a patch, cleansing foam, body cleanser, astringent, and a spray. When the formulation comes in the form of paste, cream, or gel, carriers may be used. Examples of the carrier include animal fiber, vegetable fiber, wax, paraffin, starch, tracanth, cellulose derivative, polyethylene glycol, silicone, bentonite, silica, talc, and zinc oxide. When the formulation comes in the form of powder or spray, the carrier may be lactose, talc, silica, aluminum hydroxide, calcium silicate, or polyamide. Especially in the case of powder, propellants such as chlorofluorohydrocarbon, propane/butane or dimethyl ether may be added. When the formulation comes in the form of solution or emulsion, the carrier may be a solvent, a solvating agent, or an emulsifying agent. Namely, water, ethanol, isopropanol, ethyl carbonate, ethyl acetate, benzyl alcohol, benzyl benzoate, propylene glycol, 1,3-butyl glycol oil, glycerol aliphatic esters, polyethylene glycol, or sorbitan fatty acid esters may be used. When the formulation comes in the form of suspension, liquid diluent such as water, ethanol, propylene glycol, suspending agent such as ethoxylated isostearyl alcohol, polyoxyethylene sorbitol ester, polyoxyethylene sorbitan ester, microcrystalline cellulose, aluminum metahydroxide, bentonite, agar, and tracanth may be used as carriers. When the formulation comes in the form of surfactant-containing cleansing, fatty alcohol sulfate, fatty alcohol ether sulfate, sulfosuccinic acid monoester, isethionate, imidazolinium derivative, methyltaurate, sarcosinate, fatty acid amide ether sulfate, alkylamidobetaine, fatty alcohol, fatty acid glyceride, fatty acid diethanolamide, vegetable oil, linolin derivatives, and ethoxylated glycerol fatty acid esters may be used as carriers.

Hereinbelow, the present disclosure will be described in greater detail with reference to exemplary embodiments. However, these embodiments are given by way of illustration only, and not by way of limitation.

### <Example 1> Peptide Synthesis

AR Pep1 peptide having SEQ ID NO: 1, stated in Table 1 below, is made of D-amino acids and was synthesized using the FMOC solid-phase method with an automatic peptide synthesizer (PeptrEX-R48, Peptron, Korea). The synthesized peptide was purified and analyzed by reversed-phase high-speed liquid chromatography (Prominence LC-20AB, Shimadzu, Japan) using a C18 RP column (Shiseido capcell pak), and was identified using a mass spectrometer (HP 1100 Series LC/MSD, Hewlett-Packard, USA). In other words, the synthesized peptide is composed of the amino acid sequence of SEQ ID NO: 1 (Asp-Lys-Phe) and all of the amino acids (Asp, Lys, and Phe) constituting the peptide are D-form.

**[Table 1]**

| Peptide name | SEQ ID NO: | Amino acid sequence |
|---|---|---|
| AR Pep1 | SEQ ID NO: 1 | Asp-Lys-Phe |

### <Example 2> Confirm the peptide produced in Example 1 reduces expression of the PSA by inhibiting androgen receptor activity in prostate cancer cells

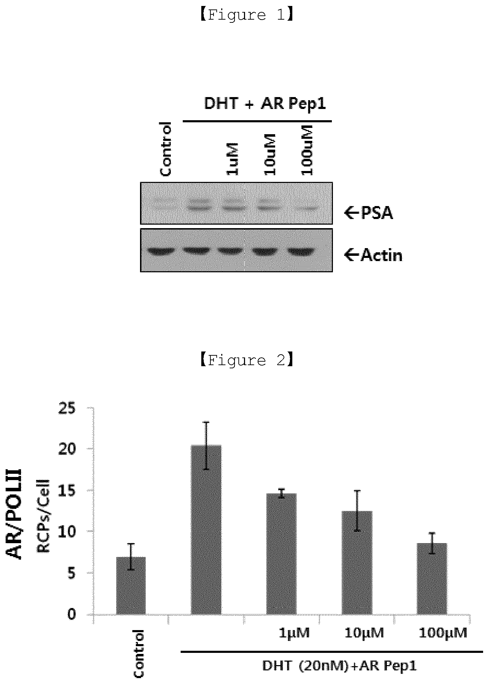

1. Prostate cancer cells (LNCaP) were prepared at a density of 5×10⁶ cells per well, treated with DHT at a concentration of 20 nM and with AR Pep1 at a concentration of 1, 10, 100 µM respectively, then cultured in an incubator at 37°C in the presence of 5% CO₂ for 24 hours. As a control group, one with no treatment at all and one treated only with DHT were used. The cancer cells were washed with PBS three times, and lysed with 1% NP40 lysis buffer (1% Nonidet P40, 0.1 M NaCl, 0.05 M tris (pH 8.0), 5 mM EDTA) containing 0.1 µM PMSP (phenylmethylsulfonyl fluoride), 1 µg/ml pepstatin A, 10 µg/ml leupeptin, 1 µg/ml aprotinin and 1mM Na₃VO₄.
2. The cell lysate was quantified using Bradford assay to prepare a protein sample for electrophoresis.
   Immunoprecipitates were subjected to 12.5% polyacrylamide gel electrophoresis, then the unfolded protein was transferred onto a nitrocellulose membrane, treated with a blocking solution (Tris-buffered saline (TBS) containing 0.05% Tween 20 and 3% bovine serum albumin) for about one hour at room temperature. Then, the membrane was suspended in TBS buffer containing Anti-PSA polyclonal antibody (Santa cruz, CA, USA) for two hours and washed with TBS buffer containing 0.05% Tween 20. Next, it was treated with horseradish peroxidase conjugated anti-rabbit IgG (Santa cruz, USA) for one hour at room temperature, washed with TBS buffer containing 0.05% Tween 20 five times, and developed using antibody detection kit(Ab frontier, Korea). The results are shown in FIG. 1. Actin was recognized using Anti-beta actin monoclonal antibody (Santa cruz, CA, USA) to confirm that the same amount of a cell lysate was electrophoresed.
3. As shown in FIG. 1, expression of prostate-specific antigen (PSA) was promoted when the prostate cancer cells were treated only with DHT, but when AR Pep1 was added with DHT, expression of the PSA was reduced, thereby confirming that AR Pep1 inhibits androgen receptor activity in prostate cancer cells.

### <Example 3> Confirm the peptide produced in Example 1 hinders physical binding of AR and POL II by inhibiting androgen receptor activity

1. Human keratinocytes (HaCaT) were prepared at a density of 4.5×10⁴ cells per well, treated with DHT at a concentration of 20 nM and with AR Pep1 at a concentration of 20 nM, 1, 10, 100 µM respectively, then cultured in an incubator at 37°C in the presence of 5% CO₂ for 24 hours. As a control, one with no treatment at all and one treated only with DHT were used. The cells in each well were washed with PBS, fixed in 2% formaldehyde for five minutes, and treated with 0.1% TritonX-100 for five minutes to enhance antibody permeability into cells. Then, Anti-AR polyclonal antibody (Santa cruz, USA) and Anti-POL II monoclonal antibody (Santa cruz, USA) were added, PLA probe was applied using In situ PLA kit (Sigma-Aldrich), and steps of hybridization, ligation, amplification, and mounting were performed.
2. Luminescence signals (PLA signals) detected from each cell were measured with a confocal laser microscope (Olympus fluoview FW1000; Olympus, Japan), physical interaction between the AR and POL II antibodies was quantified. The results are shown in Table 2.
3. As shown FIG. 2, physical interaction between the AR and POLII increases when human keratinocytes were treated with DHT, but when AR Pep1 was added with DHT, the increse in that interaction was reduced, thereby confirming that AR Pep1 inhibits androgen receptor activity. In other words, AR Pep1 inhibits androgen receptor activity, reducing the increse in interaction between the AR and POL II by DHT, and in turn alleviating skin disorders such as acne and alopecia.

### <Example 4> Animal testing regarding the effect of the peptide produced in Example 1 on prevention of hair loss

1. The back of the 7-week-old C57BL/6 mouse was depilated by waxing and injected with DHT on the neck area for five days. Same amount of sample was applied on the mouse's skin every day for another 20 days, and after that, the skin from the dorsal stripped region was harvested and was fixed in formalin fixative solution. Once formalin-fixed skin tissue was made into a paraffin block, sectioning and staining (H&E staining) were conducted, then it was observed with a light microscope and the number of hair follicles was compared. As a control group, sample 1 used PBS solution, samples 2 to 4 used PBS solutions containing 2%, 3%, 4% of AR Pep1 respectively.
2. As a result of the test, the samples 2 to 4 showed more than 100% increase in the number of hair follicles compared to the sample 1, and the number of hair follicles increased in the order of the sample 2, 3, 4.

### <Example 5> Confirm the sebum suppression effect of a cosmetics composition containing the peptide produced in Example 1 through skin patch test

1. The test was conducted on 25 men in their 30s who suffer from excessive sebum secretion. First, the forehead area was wiped with 70% ethanol and dried. Sebutape was placed on the forehead then removed and checked. Same amount of cosmetics composition was applied every morning and night for three weeks (soak a sheet in the cosmetics composition, put the sheet on the forehead for a predetermined period of time). After three weeks, the Sebutape was placed on the forehead then removed and checked. The Sebutape patches before and after applying the cosmetics composition were compared and relative decrease in sebum was confirmed. The cosmetics composition containing AR pep1, 0.02% by weight antiseptic, 0.4% by weight spices, 2% by weight glycerin, and the remaining amount (% by weight) of purified water, the cosmetics compositions 1 to 4 contain 0%, 2%, 3%, 4% by weight of AR pep1 respectively.
2. As a result of the test, when checking the forehead skin after using the cosmetics composition for three weeks, no irritation occurred, and when checking the relative reduction of sebum before and after using the cosmetics composition, no meaningful decrease in sebum was confirmed in the case that the cosmetics composition 1 which is a control group was used, whereas a significant decrease in sebum was confirmed in the case that the cosmetics compositions 1 to 4 were used. In addition, it was confirmed that the sebum reduction was becoming larger in the order of the cosmetics composition 2, 3, 4.

### <Example 6> Confirm the alopecia relief effect of a cosmetics composition containing the peptide produced in Example 1 through skin patch test

1. The test was conducted on 10 men with androgenetic alopecia with no genetic cause (It is known that androgen is an important factor in androgenetic alopecia). After applying the same amount of the cosmetics compositions 1 to 4 of Example 4 to the hair loss spot every morning and evening for 8 weeks, and after 8 weeks, the hair condition was visually checked.
2. As a result of the test, when the cosmetics composition 1 which is a control group was used, the hair did not thicken nor regrow, but when the cosmetics compositions 2 to 4 were used, it was confirmed that the hair became thicker and the number increased. Also, it was confirmed that the number increased in the order of the cosmetics composition 2, 3, 4.

In the above, the applicant described preferred embodiments of the present disclosure. It should be interpreted that such embodiments are merely examples which implement the technical idea and any modification or revision falls within the scope of the prevent disclosure if it implements the technical idea of the present disclosure, however.

## Claims

1. A peptide comprising the amino acid sequence of SEQ ID NO: 1.

2. The peptide of claim 1, wherein all of the amino acids constituting the peptide are D-amino acids; and
the peptide reduces an activity of an androgen receptor (AR).

3. The peptide of claim 1, wherein the peptide is used to alleviate skin troubles.

4. The peptide of claim 3, wherein the peptide is used to alleviate acne or alopecia.

5. A cosmetics composition comprising a peptide comprising the amino acid sequence of SEQ ID NO: 1.; and
all of the amino acids constituting the peptide are D-amino acids.

6. The cosmetics composition of claim 5, wherein the cosmetics composition has an effect of alleviating acne or alopecia.
